# EUROPEAN PATENT SPECIFICATION

(11) **EP 0 193 883 B1**
(45) Date of publication and mention of the grant of the patent: **30.06.1993**
(21) Application number: 86102594.8
(22) Date of filing: 28.02.1986
(51) Int. Cl.: C12M 1/16

(54) **Well forming apparatus**
Lochstanzgerät
Appareil pour réaliser des cavités

(30) Priority: 08.03.1985 IT 2105185 U
(43) Date of publication of application: 10.09.1986
(73) Proprietor: GRUPPO LEPETIT S.p.A., I-20159 Milano (IT)
(72) Inventor: De Ros, Angelo, I-20091 Bresso (MI) (IT); Cavenaghi, Luigi, I-20149 Milano (IT)
(74) Representative: Sgarbi, Renato

(56) References cited:
- EP-A- 0 086 706
- EP-A- 0 159 950
- GB-A- 2 067 126
- US-A- 3 631 575
- US-A- 3 863 533

## Description

The present invention relates to an apparatus which is able to form a desired number of wells in dishes or plates containing gelatinous culture media, such as agar-based media like the normal dishes or plates used for microbiological cultures and assays.

The apparatus of the invention is able to simultaneously form a desired number of wells with a limited number of simple operations. The well is formed in the agar, or other gelatinous matrix of similar consistency (hereinafter for the sake of convenience called "agar"), by the cutting action of hollow stainless steel borers which penetrate the entire depth of the agar. The plug of agar that thus fills into the distal portion of the hollow borer is removed and collected in an apposite chamber by applying aspiration (vacuum). The dish or plate with the wells formed in the agar is then ready for use, or can suitably be stored until the time of use.

A wide variety of assay and analysis methods based on the diffusion of reagents and substrates on agar or similar matrices are known. For instance, a traditionally widely used type of assays relates to the production of antibiotics by unknown microorganisms, or to the microbiological activity of a known antibiotic. In general, these methods use a microorganism susceptible to the antibiotic to be assayed, which test organism is grown in the nutrient medium that fills, or will be used to fill, the dish or plate. An appropriate solution of the test substance is then placed on the surface of the medium on the plate and, after the requisite incubation period, the diameter of the zone of inhibition produced by the test substance is measured and correlated to the concentration of the antibiotic substance.

Substantially the same technique has more recently been successfully applied to evaluation methods based on antigen-antibody reactions and formation of antigen-antibody complexes which are commonly known as immunodiffusion techniques.

Two methods are normally used for placing the test substance onto the dish or plate (i.e. the support containing the agar-based culture medium): one method includes to place onto the surface of the agar in the dish or plate a reservoir containing, or apt to contain, a solution of the test substance, such as paper discs which have been dipped in a solution of the test substance or hollow cylinders apt to contain a solution of the test substance; the other method is to place the solution of the test substance into a well preformed in the agar.

On laboratory scale, these latter wells are normally obtained by the manual use of a single hollow borer. This technique suffers from many drawbacks which includes that of having to remove the agar plug manually from the hollow borer.

EP-A 86706 describes a semiautomatic device for forming wells in an agar-like substance in a Petri dish by lowering a body having a number of hollow borers, onto the agar surface thereby penetrating the gelatinous material and subsequently removing the so formed plugs by aspiration.

GB-A 2067126 discloses a device for forming openings in a gelatinous substance in a Petri dish in which the Petri dish is lifted towards punch tools.

The present invention relates to an apparatus, particularly suitable for use on laboratory scale, which permits simultaneous boring of a desired number of wells in a base-layer of agar or material of similar consistency contained in dishes or plates, with simultaneous removal of the agar plugs which form.

The advantages deriving from the use of such an apparatus will be immediately evident to persons of ordinary skill in the art.

Such advantages include:
a) ease and rapidity of execution
b) uniformity of the dimensions of the bored wells and of their relative distances
c) considerable time-saving in the preliminary procedures called for when analyzing a large number of samples on a laboratory scale.

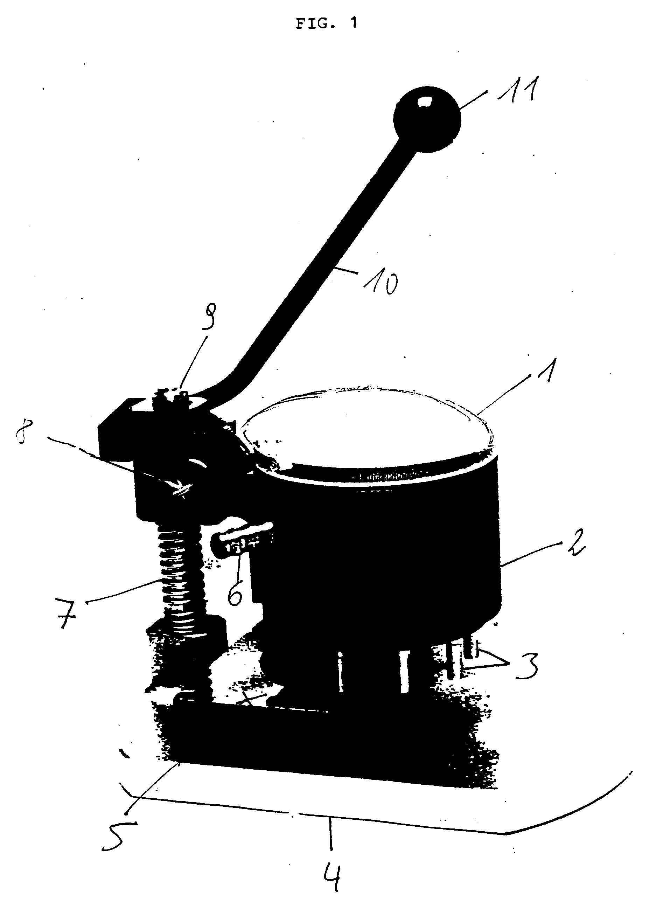

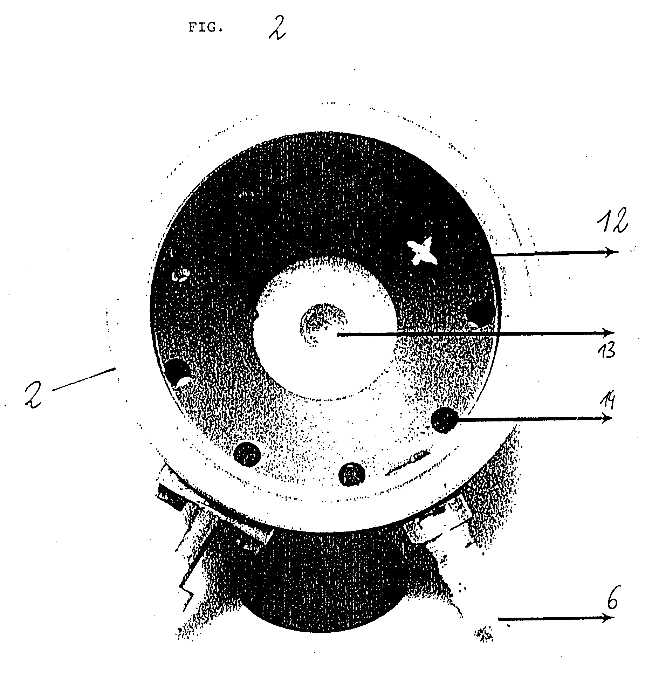

Figure 1 illustrates an example of an embodiment of the apparatus according to the invention, it includes:
a central body carrying the hollow borers, which are of predetermined dimensions and at a predetermined distance one from the other,
means for aspirating the agar plugs removed from the agar contained in the dish or plate, consisting of a chamber connected to a vacuum system and comprising a collecting portion for the agar plugs which are removed
baseplate and support and housing means for the central body
means for actuating the central body by lowering and for the automatic return to the raised position
means for positioning the dish or plate on the baseplate so as to maintain the dish or plate in a correct position beneath the central body
With reference to Figures 1, 2 and 3, the dimensions of the central body depend on those of the dish or plate that is utilized, and are such as to house the preselected number of hollow borers of predetermined dimensions and at predetermined distances. The dimensions of the hollow borers, and in particular of the distal portion thereof intended to bore out the well in the agar, depend not only on the dimensions of the dish or plate but also on the amount of the test substance (generally a solution or suspension) which they will contain; such dimensions can be selected for example from a range of internal diameters of from 1 mm to 20 mm, and preferably will be 5-9 mm. Preferably, the central body which houses the hollow borers has a truncated cone shaped internal cavity into which debouch the conduits of the proximal portions of the hollow borers, which central cavity, after it has been closed by means of a cover can act as an aspiration and collection chamber for the removed agar plugs.

The central body also presents a fitting for connection to the aspiration means, which is generally a normal central vacuum system or vacuum pump able to provide a force of aspiration sufficient to aspirate the agar plugs from the point of their formation and draw them into the collection chamber. In general, the vacuum values available in laboratories (5-50 mmHg) are adequate for this purpose. In any case, the value of the vacuum required depends on the number and dimensions of the wells simultaneously bored by the apparatus, and can readily be determined by preliminary tests on the basis of the results of the present disclosure.

The central body can advantageously be constructed from plastic material (as for example PVC), but the use of metal is also a viable possibility.

The hollow borers, on the other hand, are constructed from stainless steel, and their terminal blades are precision sharpened. As the wells are generally required to be circular, the hollow borers have as a rule a circular cross-section.

In order that the invention may be more clearly understood, preferred embodiments will be described by way of example, with reference to the accompanying drawings, in which:
Fig.1 is a schematic view showing the construction of an embodiment of the claimed well forming apparatus;
Fig.2 is a detail view of the the central body, and
Fig.3 is a detail view of the cover and cover-release means of the central body.

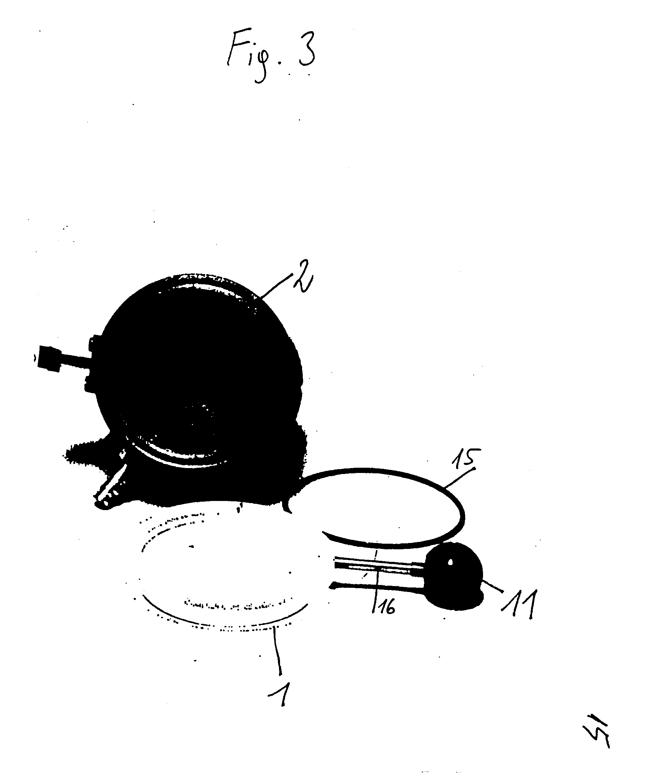

Particularly preferred for applications on laboratory scale is the apparatus shown in Figure 1, which incorporates:
(1): a transparent plastic screw-cover
(2): a central PVC body which carries the stainless steel borers (3), a fitting (6) for connection to the hollow aspiration system (vacuum pump) and an internal hollow chamber which has, in its lower portion, a preferential zone for collecting the agar plugs (aspiration cylinder)
(3): hollow stainless steel borers suitably shaped at their ends in contact with the agar so as to bore out a well without agar residues on the bottom of the plate and with falls free from unevennesses
(4): a baseplate
(5): means for positioning the dish or plate (positioner)
(6): a vacuum fitting
(7): a spring for raising the central body after actuation so as to permit the easy positioning and removal of the dishes or plates
(8): a handle for locking and unlocking the central body in order to remove it when necessary
(9): a support shaft for the central body lowering and raising means
(10): an actuating lever which is hollow, at least in its upper portion
(11): a ball-grip carrying the cover-release rod (16) (see figure 3).

Figure 2 gives a view of the interior of the central body (2). It consists of a cone shaped internal cavity (12), connected to a vacuum system via a vacuum fitting (6), comprising a collection portion (13) for the agar plugs which are removed. The proximal portions (14) of the hollow borers (3) debouch into the internal cavity.

As illustrated in Figure 3 the central body (2) is closed by means of a screw-cover (1) and a vacuum seal ring (15). The screw-cover is preferably transparent in order to permit trouble free visual inspection of the internal portion of the aspiration chamber. The screw-cover is preferably made of plastics, for example plexiglas, but can also be made of glass. Advantageously, said cover has a dead hole on its edge to which a cover-release means may be adapted, which can cooperate in opening the cover.

This cover-release means, is conveniently formed of the ball-grip (11) of the actuating lever 10, which carries a rod (16) of suitable length and has a size such that it can be housed into said lever which, at least in its upper portion, is consequently hollow.

According to a feature of the present invention relating to an embodiment thereof suitable for use on laboratory scale, the central body is actuated by means of a lever which, when depressed, causes the lowering of the central body and thus the penetration of the agar by the cutting end of the hollow borers so as to bore out wells of predetermined dimensions. The return of the lever and thus of the central body to its raised (or rest) position can in this case be effected by the action of a mechanical spring which extends after being compressed by the depression of the actuating lever.

As will be apparent to persons of ordinary skill in the art, many components of the apparatus according to the invention that has been illustrated above by way of example can be replaced by functionally equivalent component parts. For example, the mechanical spring heretofore described can be replaced by any equivalent technical means, such as a hydraulic or pneumatic means.

The lowering arm is likewise simply a convenient means for embodiments of the apparatus suitable for use on a small scale, while other equivalent systems can fulfill the same function and may also prove preferably when the apparatus is used on an industrial scale, as for example when the number of operations repeated daily is such as to require an electronically controlled system for actuating a device for lowering the central body and returning it to a raised position above the baseplate of said body through the intermediary of a motor. In such a case, the operation of positioning the dish or plate and of collecting the treated plates is preferably automated and controlled by an electronic system.

The dish or plate positioner facilitates the centering of the dish or plate in the correct position so that the position of the wells is pre-established. The dimension and form of the dish or plate positioner naturally depend on the dimension and form of the dish or plate employed. Preferably, said positioner is constructed from plastic materials or metal, and is secured to the baseplate and/or support shaft in such a manner as to allow the plate to be suitably replaced as needed.

For embodiments of the apparatus of the invention intended for the treatment of a given type of dish or plate, said positioner can also be fixed to the baseplate instead of being removable.

As stated previously, the number of wells and their size depend not only on the size of the dish or plate, but also on the specific use for which the dish or plate is intended, and, ultimately, on the amounts and the diffusion properties of the used reagents.

For circular dishes or plates it is generally preferable to bore a single series of wells along the circumference at a sufficient distance from the edge, so that the central body will also be such as to carry the hollow borers arranged along a circumference. If the specific application so requires, provision can be made for a central well or a more inwardly positioned circle of wells, or for any other arrangement, with appropriate modification of the geometry of the central body and the disposition of the hollow borers in it. In the case of square dishes or plates, it is generally preferred to operate with wells disposed in several rows. In such a case, the central body will carry the hollow borers disposed in rows instead of in circles.

The dimensions of the dishes or plates suitable for treatment using the apparatus of the invention vary, and include those available on the market.

Examples of dishes or plates are circular dishes or plates having a diameter of from 35 mm to 145 mm, and square dishes or plates having sides of length ranging from 100 mm to 230 mm.

Square dishes or plates with a size larger than, but multiples of, the size of the central body or an apparatus of the invention can also be treated by successively positioning them under said apparatus of the invention.

## Claims

1. An apparatus for simultaneously forming a desired number of wells in dishes or plates containing a gelatinous matrix consisting of:
a central body (2) with a transparent screw-cover (1), which contains an internal cavity (12),
wherein said central body carries a selected number of hollow stainless steel borers (3), positioned at the underside of the central body in a predetermined distance one from the other, which borers debouch into said internal cavity, and has a fitting (6), to connect the internal cavity with an aspiration means;
a base plate (4);
means for connecting said baseplate to said central body (2), thereby providing support for said central body;
means for positioning and keeping stationary said plate or dish (5) on the baseplate;
means (10) for lowering the central body towards said stationary dish or plate, without any motion perpendicular thereto, and
means (7) for raising up the central body from said dish or plate, without any motion perpendicular thereto.

2. An apparatus as described in claim 1, comprising:
(1) a transparent plastic screw-cover
(2) a central PVC body which carriers the stainless steel borers (3), a fitting (6) for connection to the hollow aspiration system and an internal hollow chamber (12) which has, in its lower portion, a preferential zone (13) for collecting the agar plugs
(3) hollow stainless steel borers suitably shaped at their ends in contact with the agar so as to bore out a well without agar residues on the bottom of the plate and with falls free from unevennesses
(4) a baseplate
(5) means for positioning the dish or plate
(6) a vacuum fitting
(7) a spring for raising the central body after actuation so as to permit the easy positioning and removal of the dishes or plates
(8) a handle for locking and unlocking the central body in order to remove it when necessary
(9) a support shaft for the central body lowering and raising means
(10) an actuating lever, which is hollow, at least in its upper portion
(11) a ball-grip carrying the cover-release rod (16).

3. Method for simultaneously forming a desired number of wells in dishes or plates containing a gelatinous matrix by using the apparatus of claim 1, wherein :
a) the central body is actuated by means of a lever which, when depressed, causes the lowering of the central body and thus the penetration of the agar by the cutting end of the hollow borers so as to bore out wells of predetermined dimensions;
b) the return of the lever and thus of the central body to its raised (or rest) position is effected by the action of a mechanical spring which extends after being compressed by the depression of the actuating lever; and
c) the agar plugs, removed from the dishes or plates, are aspirated by a vacuum system and transported to a collecting chamber in the internal cavity of the central body into which the proximal portions of the hollow borers debouch.

## Patentansprüche

1. Vorrichtung zum gleichzeitigen Formen einer erwünschten Anzahl von Bohrungen in Schalen oder Platten, die eine gelatineartige Matrix enthalten, bestehend aus:
einem zentralen Körper (2) mit einer transparenten Schraubabdeckung (1), der einen Innenhohlraum (12) enthält,
wobei der zentrale Körper eine gewählte Anzahl von hohlen rostfreien Stahlbohrern (3) trägt, die an der Unterseite des zentralen Körpers in einem vorbestimmten Abstand voneinander angeordnet sind, wobei die Bohrer in den Innenhohlraum münden, und mit einem Anschluß (6) zum Anschließen des Innenhohlraumes an Saugmittel;
einer Grundplatte (4);
Mitteln zum Verbinden der Grundplatte mit dem zentralen Körper (2), wodurch eine Abstützung für den zentralen Körper geschaffen wird;
Mitteln zum Positionieren und Stationärhalten der Platte oder der Schale (5) auf der Grundplatte;
Mitteln (10) zum Absenken des zentralen Körpers auf die stationäre Schale oder Platte ohne irgendeine Bewegung senkrecht dazu, und
Mitteln (7) zum Anheben des zentralen Körpers von der Schale oder Platte ohne irgendeine Bewegung senkrecht dazu.

2. Vorrichtung nach Anspruch 1, umfassend:
(1) eine transparente Kunststoff-Schraubabdeckung
(2) einen zentralen PVC-Körper, welcher die rostfreien Stahlbohrer (3), einen Anschluß (6) zum Anschließen an das hohle Saugsystem und eine innere hohle Kammer (12) trägt, die in ihrem unteren Abschnitt eine bevorzugte Zone (13) zum Sammeln der Agarstopfen aufweist
(3) hohle rostfreie Stahlbohrer, die an ihren mit dem Agar in Kontakt stehenden Enden geeignet geformt sind, so daß sie eine Bohrung ohne Agarrückstände am Boden der Platte herausbohren, und mit distalen Abschnitten, die frei von Unregelmäßigkeiten sind
(4) eine Grundplatte
(5) Mittel zum Positionieren der Schale oder Platte
(6) einen Sauganschluß
(7) eine Feder zum Anheben des zentralen Körpers nach der Betätigung, damit ein leichtes Positionieren und Entfernen der Schalen oder Platten gestattet wird
(8) einen Handgriff zum Verriegeln und Entriegeln des zentralen Körpers, um diesen bei Bedarf zu entfernen
(9) einen Tragschaft für die Absenk- und Anhebemittel des zentralen Körpers
(10) einen Betätigungshebel, der zumindest in seinem oberen Abschnitt hohl ist
(11) einen Kugelgriff, welcher die Abdeckungsfreigabestange (16) trägt.

3. Verfahren zum gleichzeitigen Formen einer erwünschten Anzahl von Bohrungen in Schalen oder Platten, die eine gelatineartige Matrix enthalten, unter Verwendung der Vorrichtung nach Anspruch 1, bei welchem:
a) der zentrale Körper mittels eines Hebels betätigt wird, der beim Niederdrücken das Absenken des zentralen Körpers und somit das Eindringen des Schneidendes der hohlen Bohrer in das Agar verursacht, um Bohrungen vorbestimmter Abmessungen herauszubohren;
b) das Rückstellen des Hebels und somit des zentralen Körpers in seine angehobene Stellung (oder Ruhestellung) durch die Wirkung einer mechanischen Feder bewirkt wird, die sich ausdehnt, nachdem sie durch Niederdrücken des Betätigungshebels zusammengedrückt worden ist; und
c) die von den Schalen oder Platten entfernten Agarstopfen durch ein Saugsystem angesaugt und in eine Sammelkammer im Innenhohlraum des zentralen Körpers transportiert werden, in welchen die proximalen Abschnitte der hohlen Bohrer münden.

## Revendications

1. Appareil pour former simultanément un nombre souhaité de cavités dans des boîtes ou des plaques contenant une matrice gélatineuse comprenant :
un corps central (2) ayant un couvercle fileté transparent (1), qui contient une cavité interne (12),
dans lequel ledit corps central porte un nombre choisi de forets creux en acier inoxydable (3), placés au niveau de la surface inférieure du corps central à une distance prédéterminée l'un de l'autre, lesquels forets débouchent dans ladite cavité interne, et comporte un raccord (6) pour relier la cavité interne à un moyen d'aspiration ;
une plaque de base (4) ;
des moyens pour relier ladite plaque de base audit corps central (2), de façon à fournir un support audit corps central ;
un moyen pour positionner et garder en place ladite plaque ou boîte (5) sur la plaque de base ;
un moyen (10) pour abaisser le corps central vers ladite boîte ou plaque fixe, sans faire de mouvement perpendiculaire pour cela, et
un moyen (7) pour relever le corps central de ladite boite ou plaque, sans faire de mouvement perpendiculaire pour cela.

2. Appareil selon la revendication 1, comprenant :
(1) un couvercle fileté en plastique transparent
(2) un corps central en PVC qui porte les forets en acier inoxydable (3), un raccord (6) pour relier le système d'aspiration creux et la chambre creuse interne (12) qui comporte, dans sa partie inférieure, une zone préférentielle (13) pour collecter les bouchons d'agar-agar
(3) des forets creux en acier inoxydable convenablement mis en forme à leurs extrémités de contact avec l'agar-agar de façon à creuser une cavité sans résidu d'agar-agar sur le fond de la plaque et ayant des bords exempts d'irrégularités.
(4) une plaque de base
(5) des moyens de positionnement de la boîte ou de la plaque
(6) un raccord à vide
(7) un ressort pour élever le corps central après actionnement de façon à permettre le placement et l'enlèvement aisé des boîtes ou des plaques.
(8) une poignée pour bloquer ou débloquer le corps central afin de l'enlever quand nécessaire.
(9) un axe support pour les moyens d'élévation et de descente du corps central
(10) un levier d'actionnement qui est creux, du moins dans sa partie supérieure
(11) une poignée à rotule portant la tige de libération du couvercle (16) .

3. Procédé pour former simultanément un nombre souhaité de cavités dans des boîtes ou dans des plaques contenant une matrice gélatineuse en utilisant l'appareil de la revendication 1, dans lequel :
a) le corps central est actionné au moyen d'un levier qui, lorsque détendu, provoque l'abaissement du corps central et ainsi la pénétration de l'agar-agar par l'extrémité coupante des forets creux de façon à creuser des cavités de dimensions prédéterminées;
b) le retour du levier et ainsi du corps central vers sa position élevée (ou position de repos) est effectué par l'action d'un ressort mécanique qui s'allonge après avoir été compressé par la dépression du levier d'actionnement ; et
c) les bouchons d'agar-agar, retirés des boîtes ou des plaques, sont aspirés par un système de vide et transportés vers une chambre de collecte dans la cavité interne du corps central dans laquelle les parties proximales des forets creux débouchent.
